# EUROPÄISCHE PATENTSCHRIFT

(11) **EP 0 536 577 B1**
(45) Veröffentlichungstag und Bekanntmachung des Hinweises auf die Patenterteilung: **05.01.2000**
(21) Anmeldenummer: 92116079.2
(22) Anmeldetag: 21.09.1992
(51) Int. Cl.: H05B 41/36, H01J 61/02

(54) **Vorrichtung mit einer eine Entadungsröhre enthaltenden Lampe und Verfahren zur Steuerung dieser Vorrichtung**
Apparatus with a lamp comprising a discharge tube and method of control of this apparatus
Système avec une lampe munie d'un tube à décharge et méthode à commande de tel appareil

(30) Priorität: 10.10.1991 DE 4133614
(43) Veröffentlichungstag der Anmeldung: 14.04.1993
(73) Patentinhaber: Heraeus Noblelight GmbH, 63450 Hanau (DE)
(72) Erfinder: Waldhauer, Lothar, W-8044 Lohhof (DE)
(74) Vertreter: Staudt, Armin Walter

(56) Entgegenhaltungen:
- EP-A- 0 210 626
- EP-A- 0 444 591
- WO-A-90/06040
- DE-A- 2 718 527
- US-A- 3 862 414
- US-A- 4 529 912
- PATENT ABSTRACTS OF JAPAN vol. 6, no. 55 (E-101)10. April 1982, & JP-A-56 167236 ( TOSHIBA CORP ) 22. Dezember 1981

## Beschreibung

Die Erfindung betrifft eine Vorrichtung mit einer Lampe gemäß Oberbegriff von Anspruch 1 sowie ein Verfahren zur Steuerung dieser Vorrichtung gemäß Oberbegriff von Anspruch 1, die eine Entladungsröhre umfaßt, in der ein Gasgemisch und strahlungsemittierende Substanzen eingeschlosssen sind. Eine derartige Vorrichtung ist aus JP-A-56 167 236 mit englischer Zusammenfassung in "Patent Abstracts of Japan", Band 6, Nr. 55 (E-101) vom 10.4.1982 bekannt.

Entladungsröhren enthalten ein durch Gasentladung zur Strahlungsemission angeregtes Gasgemisch, wobei Leuchtstoffröhren zusätzlich einen auf das Innere der Entladungsröhre aufgebrachten Leuchtstoff besitzen, der die kurzwellige elektromagnetische Strahlung aus der Gasentladung in längerwellige Strahlung umwandelt.

Beispielsweise kommen bei Lampen, durch deren Bestrahlung eine erhöhte Pigmentierung der Haut oder eine therapeutische Wirkung auf erkrankte Hautbezirke erzielt werden soll, Gasentladungsröhren zum Einsatz, deren abgestrahltes Spektrum im ultravioletten Bereich Intensitätsmaxima aufweist. Eine Bestrahlung mit die menschliche Haut schädigenden kurzwelligen UVB-Strahlen muß dabei vermieden werden. Dies kann durch Optimierung des in der Entladungsröhre vorhandenen Gasgemisches mit seinen strahlungsemittierenden Substanzen und durch Ausfilterung der UVB-Strahlung geschehen.

UV-strahlende Entladungsröhren arbeiten mit der bekannten Quecksilberdampfhochdruckentladung, wobei dem Röhreninneren Metallhalogenide zugesetzt sind, um die integrale Intensität im UVA-Bereich (315 - 380 nm) gegenüber derjenigen im UVB-Bereich (280 - 315 nm) zu erhöhen (DE-27 18 735 C2).

Allgemein ist bei Lampen mit Entladungsröhren, die in kosmetischen, medizinischen und technischen Gebieten (UV-Härtung, graphische Reproduktionstechnik) eingesetzt werden und in einem bestimmten durch die jeweilige Anwendung festgelegten Spektralbereich ihre höchste Emissionsintensität besitzen sollen, zu fordern, daß die Strahlungsintensität in den übrigen Spekralbereichen überwiegend möglichst gering gehalten ist. Diese Forderung wird jedoch nicht von allen auf dem Markt erhältlichen Entladungsröhren erfüllt.

Gesundheitsschäden oder Fehler bei technischen Herstellungsverfahren können deshalb die Folge sein.

Weitere Probleme können auftreten, wenn Entladungsröhren über den durch die vom Hersteller angegebene Lebensdauer begrenzten Zeitraum hinaus verwendet werden. Die spektrale Intensität der Entladungsröhre hat dann erheblich abgenommen oder sich in andere Spektralbereiche verschoben.

Ausbleibende Erfolge oder unbefriedigende Ergebnisse bei der Anwendung solcher Entladungsröhren sind in der Regel das Resultat.

Aufgabe vorliegender Erfindung ist es deshalb, eine verbesserte Entladungsröhre zu entwickeln, die einen kontrollierten Einsatz ermöglicht, bei dem die obengenannten Nachteile vermieden werden.

Diese Aufgabe wird erfindungsgemäß durch die Merkmale der Vorrichtung gemäß Anspruch 1 und durch das Verfahren gemäß Anspruch 3 gelöst.

Selbstverständlich wird man darauf zu achten haben, daß diese Emissionslinie(n) keinen nachteiligen Einfluß bei der jeweiligen Bestrahlungsanwendung ausüben.

Die weitere strahlungsemittierende Substanz wird nach zwei Gesichtspunkten ausgewählt: Zum einen soll sie erfindungsgemäß in demjenigen Spektralbereich, in dem das durch die übrigen Substanzen vorgegebene Spektrum eine niedrige Intensität besitzt, eine oder mehrere Emissionslinie(n) aufweisen, zum anderen darf durch diese Emissionslinie(n) die Qualität der Bestrahlung mit der Entladungsröhre nicht oder kaum beeinflußt werden.

Die erfindungsgemäße weitere strahlungsemittierende Substanz kann dem Gasgemisch im Inneren der Entladungsröhre oder bei Leuchtstoffröhren auch dem auf der Innenwand der Entladungsröhre befindlichen Leuchtstoff zugesetzt werden.

Die erfindungsgemäße Entladungsröhre besitzt in ihrem Spektrum eine Markierung in Form der Emissionslinie(n) der weiteren strahlungsemittierenden Substanz.

Durch diese Markierung wird es möglich, bestimmte Entladungsröhren, die beispielsweise erhöhten Qualitätsanforderungen entsprechen müssen, von anderen zu unterscheiden. Solche Qualitätsanforderungen werden beispielsweise in der Reproduktionstechnik oder in der medizinischen UV- oder IR-Bestrahlung an die Entladungsröhren gestellt. Durch die erfindungsgemäße Markierung lassen sich dann solche hochqualitativen Entladungsröhren von äußerlich gleichen, d.h. ebenfalls in die Bestrahlungslampen einsetzbaren, aber qualitativ minderwertigen Entladungsröhren unterscheiden. Damit können Fehler bei technischen Herstellungsverfahren und Gesundheitsschäden bei der medizinischen oder kosmetischen Anwendung vermieden werden.

Darüber hinaus wird bei der erfindungsgemäßen Entladungsröhre eine Aussage über ihre Betriebszeit möglich. Im Laufe des Betriebs wird naturgemäß nicht nur die Intensität des durch die übrigen Substanzen vorgegebenen Spektrums abnehmen, sondern auch die der Emissionslinie(n) der weiteren strahlungsemittierenden Substanz. Die Intensität dieser Linie(n) läßt sich demnach mit der Betriebsdauer der Entladungsröhre korrelieren. Nach entsprechender Eichung kann aus einer Messung der Intensität dieser Emissionslinie(n) eine Aussage über die Betriebsdauer der Entladungsröhre gemacht werden.

Eine weitere Ausgestaltung besteht darin, daß in der Entladungsröhre eine weitere strahlungsemittierende Substanz enthalten ist, die im sichtbaren und/oder infraroten Spektralbereich mindestens eine Emissionslinie besitzt, während die übrigen Substanzen überwiegend im ultravioletten Spektralbereich Strahlung emittieren.

Hierbei ist beispielsweise an den Einsatz solcher erfindungsgemäßen Entladungsröhren im medizinischen oder kosmetischen Bereich gedacht. Die dort eingesetzten Entladungsröhren können ohne weiteres eine Emissionslinie im sichtbaren oder infraroten Spektralbereich besitzen, ohne daß die Anwendbarkeit dadurch eingeschränkt würde. Beispielsweise würde eine solche Emissionlinie im sichtbaren Bereich lediglich die Farbe des Strahlungsfeldes der Entladungsröhren verändern.

Erfindungsgemäße Entladungsröhren, deren Füllgaszusammensetzung auf eine erhöhte UVA-Emission und auf eine stark reduzierte Strahlungsintensität im restlichen Spektralbereich, insbesondere im UVB, hin optimiert wurden, lassen sich nun von qualitativ minderwertigen, in die gleichen Bestrahlungslampen einsetzbaren, aber unter Umständen gesundheitsgefährdenden Entladungsröhren unterscheiden.

Gleichzeitig kann, wie bereits erwähnt, die Betriebszeit der UV-Entladungsröhre überwacht werden, so daß ein Überschreiten ihrer Lebensdauer verhindert werden kann.

Hierbei erweist es sich als vorteilhaft, wenn zusätzlich zu den Substanzen, die überwiegend im ultravioletten Spektralbereich Strahlung emittieren, als weitere strahlungsemittierende Substanz ein oder mehrere Alkalimetall(e) und/oder ein oder mehrere Halogenid(e) von Alkalimetall in der Entladungsröhre enthalten ist.

Metalle werden aufgrund ihrer ungünstigen Verdampfungseigenschaften meist in Halogenidform den Entladungsröhren zugesetzt. Erfindungsgemäße Entladungsröhren, die im UV-Bereich den Hauptteil ihrer Strahlungsintensität emittieren, enthalten vorteilhafterweise ein oder mehrere Alkalimetalle, insbesondere Lithium, Cäsium oder Natrium, bzw. deren Halogenide. Die genannten Metalle besitzen im sichtbaren und infraroten Spektralbereich Emissionslinien und eigenen sich deshalb als weitere strahlungsemittierende Substanz in einer erfindungsgemäßen UV-Entladungsröhre.

Es sei jedoch darauf hingewiesen, daß auch andere Metalle oder ganz allgemein chemische Verbindungen gefunden werden können, die als weitere strahlungsemittierende Substanz wirksam sein können. Bei erfindungsgemäßen UV-Entladungsröhren sei auch auf Thallium hingewiesen, das im grünen sichtbaren Spektralbereich bei 535 nm eine starke Emissionslinie besitzt.

Eine Vorrichtung zum Einsatz einer erfindungsgemäßen Entladungsröhre in einer Lampe sieht zweckmäßigerweise vor, daß im Strahlungsfeld der Entladungsröhre ein optischer Detektor angebracht ist, dessen Ausgangssignal mit der Intensität einer oder mehrerer Emissionslinie(n) der in der Entladungsröhre enthaltenen weiteren strahlungsemittierenden Substanz korreliert ist.

Das Ausgangssignal des optischen Detektors läßt sich dabei z.B. auf eine numerische Anzeige übertragen, die beim Betrieb der Lampe die Existenz und die Höhe der Intensität der Emissionslinie(n) der weiteren strahlungsemittierenden Substanz anzeigt. Wird in eine erfindungsgemäße Vorrichtung mit einer Lampe eine andere als die erfindungsgemäße Entladungsröhre eingesetzt oder eine, deren Lebensdauer überschritten ist, zeigt das Ausgangssignal des Detektors entsprechendes an. Dabei kann dieses Ausgangssignal auch in ein optisches oder akustisches Signal gewandelt werden.

Vorteilhafterweise steht das Ausgangssignal des optischen Detektors in Wirkverbindung zu der Steuereinrichtung der Lampe.

Dadurch ist es möglich, den Betrieb der Lampe zusätzlich durch das Ausgangssignal des Detektors zu steuern. Steigt beispielsweise der Pegel dieses Ausgangssignals mit wachsender Intensität der Emissionslinie(n) an, kann der Betrieb der Lampe dadurch gesteuert werden, daß, wenn der Pegel eine bestimmte Mindesthöhe, die mit der Lebensdauer der Entladungsröhre korreliert ist, unterschreitet, oder wenn überhaupt kein Pegel vorhanden ist, die Lampe abgeschaltet wird, da nicht für eine genügend hohe Bestrahlungsqualität und -sicherheit garantiert werden kann.

Zweckmäßigerweise wird also beim Unterschreiten eines vorgegebenen Pegels des Ausgangssignals des optischen Detektors die Lampe mit der Entladungsröhre außer Betrieb genommen.

Im folgenden soll ein Ausführungsbeispiel die Erfindung näher erläutern.

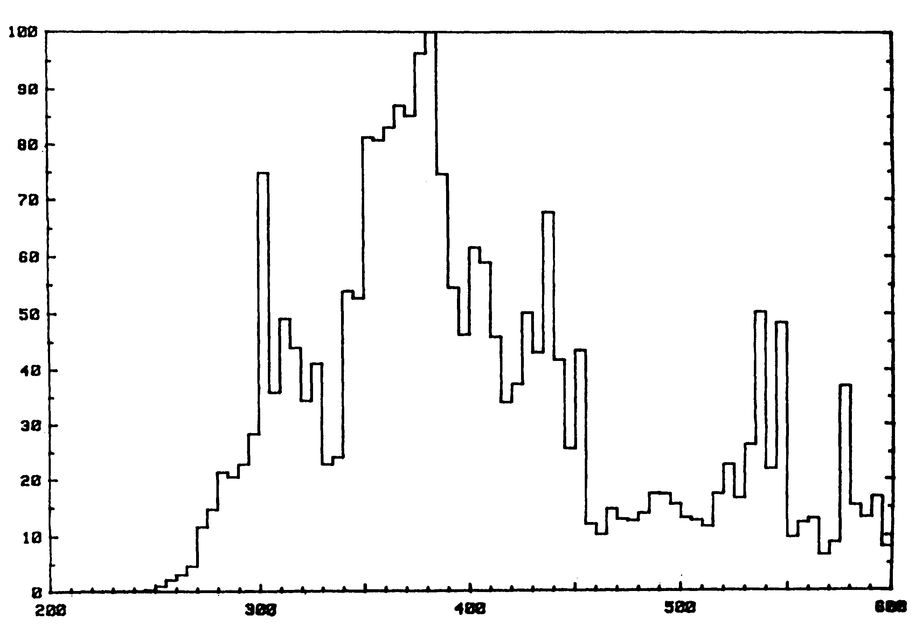

In der einzigen Figur ist das Spektrum eines sogenannten Eisenstrahlers, wie er zur UV-Bestrahlung verwendet wird, dargestellt. Aufgetragen ist die Intensität in relativen Einheiten gegen die Wellenlänge in nm-Einheiten der von der Entladungsröhre emittierten Strahlung.

Die Entladungsröhre enthält als Zündgase die Edelgase Krypton und Argon und weiterhin im wesentlichen Quecksilber und Eisen bzw. deren Halogenide (z.B. Eisenjodid) zur erhöhten Strahlungsausbeute im ultravioletten Spektralbereich. Die Leistungsaufnahme der Entladungsröhre beträgt 2 kW, der Strahlungsfluß im UVA-Bereich über 500 W.

Ersichtlich ist aus der Zeichnung eine hohe Intensität im UVA-Bereich zwischen 320 und 390 nm und eine abnehmende Intensität im UVB-Bereich zwischen 280 und 320 nm. Diese Restintensität wird durch spezielle Filter absorbiert. Unterhalb 250 nm wird emittiertes Licht aus der Gasentladung vom Quarzkolben der Entladungsröhre absorbiert. Im sichtbaren Spektralbereich besitzt die Entladungsröhre Bereiche niedriger Intensität zwischen Blau und Grün (450 - 530 nm), zwischen Grün und Gelb (550 - 580 nm) und im Roten (ab 590 nm) sowie im Infraroten. Letzterer Bereich ist wegen seines geringen Intensitätsverlaufs nicht mehr dargestellt.

Die erfindungsgemäße Entladungsröhre besitzt im wesentlichen das gleiche Spektrum, bis auf den Unterschied, daß in einem der genannten Bereiche niedriger Intensität eine oder mehrere Emissionslinie(n) durch die weitere strahlungsemittierende Substanz auftreten.

Als weitere strahlungsemittierende Substanz wird beispielsweise Natrium gewählt. Die gelben Na-Linien bei 589 und 589,6 nm liegen in einem günstigen Spektralbereich des durch die übrigen Substanzen vorgegebenen UV-Spektrums. Im übrigen wird die Wahl der weiteren strahlungsemittierenden Substanz auch von der spektralen Empfindlichkeit des Detektors abhängen.

Auch Lithium mit seinen roten Linien bei 610,4 bzw. 670,8 nm oder Cäsium mit seinen Emissionslinien im Roten und Infraroten (672,3 und 697,3 und 801,6 und 807,9 nm) oder Thallium mit seiner grünen Linie bei 535 nm eignen sich ebenso wie Natrium als Zusatz.

In unmittelbarer Nähe der erfindungsgemäßen Entladungsröhre ist in ihrem Strahlungsfeld ein optischer Detektor angebracht. Als Detektor wird eine Photodiode verwendet, der spezielle optische Filter vorgeschaltet sind, die nur einen schmalen Wellenlängenbereich, in dem die Emissionslinie(n) der weiteren strahlungsemittierenden Substanz liegen, durchlassen. Das elektrische Ausgangssignal des Detektors ist proportional zur integralen Intensität in diesem durchgelassenen schmalen Spektralbereich. Dieses Ausgangssignal wird mit der Steuereinrichtung der Lampe derart in Verbindung gebracht, daß beim Unterschreiten eines Mindestpegels des Ausgangssignals die Lampe automatisch abgeschaltet wird.

Der Mindestpegel des Ausgangssignals wird nach der noch vorhandenen Intensität des detektierten Spektralbereichs nach Ablauf der Lebensdauer der Entladungsröhren bestimmt. Die Lebensdauer beträgt in diesem Ausführungsbeispiel etwa 500 Betriebsstunden.

Durch dieses Verfahren wird es möglich, daß nur erfindungsgemäße Entladungsröhren, deren Lebensdauer nicht überschritten ist, in einer ultravioletten Bestrahlungslampe zum Einsatz kommen können. Der versehentliche Betrieb von äußerlich gleichen, von ihrer spektralen Zusammensetzung jedoch ungeeigneten Entladungsröhren, die einen ausbleibenden Erfolg oder auch Gesundheitsschäden bei der UV-Bestrahlung hervorrufen können, bleibt nunmehr ausgeschlossen.

## Patentansprüche

1. Vorrichtung mit einer Lampe, die eine Entladungsröhre umfaßt, in der ein Gasgemisch und strahlungsemittierende Substanzen eingeschlossen sind, und die eine weitere strahlungsemittierende Substanz enthält, die mindestens eine Emissionslinie besitzt, und mit einem optischen Detektor, der im Strahlungsfeld der Lampe angebracht, und dessen Ausgangssignal mit der Intensität einer oder mehrerer Emissionslinie(n) der weiteren strahlungsemittierenden Substanz korreliert ist, dadurch gekennzeichnet, die mindetsens eine Emissionslinie der weiteren strahlungsemittierenden Substanz als Markierung zur Lampenunterscheidung wirkt, indem sie einerseits in einem Spektralbereich niedriger Intensität des durch die übrigen Substanzen vorgegebenen Spektrums liegt, und andererseits das durch die übrigen Substanzen vorgegebene Spektrum nicht oder kaum beeinflußt.

2. Vorrichtung nach Anspruch 1, dadurch gekennzeichnet, daß das Ausgangssignal des optischen Detektors in Wirkverbindung zu der Steuereinrichtung der Lampe steht.

3. Verfahren zur Steuerung einer Vorrichtung nach Anspruch 2, dadurch gekennzeichnet, daß beim Unterschreiten eines vorgegebenen Pegels des Ausgangssignals des optischen Detektors die Lampe mit der Entladungsröhre außer Betrieb genommen wird.

## Claims

1. Apparatus with a lamp which includes a discharge tube in which are contained a gas mixture and radiation-emitting substances, and which contains an additional radiation-emitting substance having at least one emission line, and with an optical detector which is mounted in the radiation field of the lamp and whose output signal is correlated with the intensity of one or more emission line(s) of the additional radiation-emitting substance, characterised in that the at least one emission line of the additional radiation-emitting substance acts as a marking for lamp differentiation, by the fact that on the one hand it is within a low-intensity spectral range of the spectrum predetermined by the remaining substances, and on the other hand does not affect or hardly affects the spectrum predetermined by the remaining substances.

2. Apparatus according to claim 1, characterised in that the output signal of the optical detector is operatively connected to the control device of the lamp.

3. Method for the control of an apparatus according to claim 2, characterised in that, on falling below a predetermined level of the output signal of the optical detector, the lamp with the discharge tube is turned off.

## Revendications

1. Dispositif avec une lampe, qui comprend un tube à décharge dans lequel sont enfermés un mélange gazeux et des substances radiantes et qui contient une substance supplémentaire radiante qui possède au moins une ligne d'émission et avec un détecteur optique qui est placé dans le champ de rayonnement de la lampe et dont le signal de sortie coincïde avec l'intensité d'une ou de plusieurs lignes d'émission de l'autre substance radiante, caractérisé en ce qu'au moins une ligne d'émission de l'autre substance radiante sert de repère pour différencier les lampes, dans le fait que d'une part elle se trouve dans une zone spectrale de faible intensité du spectre prédéterminée par les substances restantes et, d'autre part, n'influence pas ou à peine le spectre prédéterminé par les substances restantes.

2. Dispositif selon la revendication 1, caractérisé en ce que le signal de sortie du détecteur optique est relié au dispositif de commande de la lampe.

3. Procédé de commande d'un dispositif selon la revendication 2, caractérisé en ce que pour une valeur inférieure au niveau prédéterminé du signal de sortie du détecteur optique, la lampe avec le tube à décharge est mise hors service.
